# NEUE EUROPÄISCHE PATENTSCHRIFT

(11) **EP 1 771 226 B2**
(45) Veröffentlichungstag und Bekanntmachung des Hinweises auf die Entscheidung über den Einspruch: **09.03.2016**
(45) Hinweis auf die Patenterteilung: 08.10.2008
(21) Anmeldenummer: 05744669.2
(22) Anmeldetag: 18.05.2005
(51) Int. Cl.: A61Q 5/06, A61Q 5/10, A61K 8/02, A61K 8/04, A61K 8/11, A61K 8/73, A61K 8/25

(54) **MEHRKOMPONENTEN-KIT UND VERFAHREN ZUM FÄRBEN VON KERATINFASERN**
MULTICOMPONENT KIT AND METHOD FOR DYEING KERATIN FIBRES
KIT MULTICOMPOSANT ET PROCEDE DE COLORATION DE FIBRES KERATINIQUES

(30) Priorität: 30.07.2004 DE 102004037105
(43) Veröffentlichungstag der Anmeldung: 11.04.2007
(73) Patentinhaber: Wella GmbH, 65825 Schwalbach am Taunus (DE)
(72) Erfinder: SCHMENGER, Jürgen, 64331 Weiterstadt (DE); BRAUN, Petra, 64839 Münster (DE)
(74) Vertreter: Boubel, Thomas
(86) Internationale Anmeldenummer: PCT/EP2005/005400
(87) Internationale Veröffentlichungsnummer: WO 2006/012935

(56) Entgegenhaltungen:
- EP-A- 0 148 681
- EP-A1- 0 560 088
- WO-A1-97/39727
- WO-A1-03/074014
- DE-A1- 4 233 874
- DE-A1- 10 230 414
- DE-A1- 10 260 880
- DE-A1- 19 548 291
- US-A- 4 566 875
- US-A- 5 261 926
- US-B1- 6 440 175

## Beschreibung

Gegenstand der vorliegenden Erfindung ist ein Mehrkomponenten-Kit sowie ein Verfahren zum Färbem von Keratinfasern, insbesondere menschlichen Haaren.

Farbstoffe, die üblicherweise für die Färbung von Keratinfasern eingesetzt werden, sind sogenannte Direktzieher, Nitrofarbstoffe und Pigmentfarbstoffe oder Oxidationsfarbstoffe, die als farblose Entwickler-/Kupplervorstufen vorliegen.

Nach dem Stand der Technik hergeste35llte Färbemittel werden in üblichen Darreichungsformen angeboten. Diese Darreichungsformen variieren von flüssigen bis zu creme- und wachsartigen Produkten. Auch Aerosole, beispielsweise sogenannte Schaumhaarfarben, finden Anwendung. Ebenso sind nach dem Stand der Technik pulverförmige Farben auf dem Markt, die vor der Anwendung mit einem wässrigen Medium gemischt werden müssen. Beispiele für pulverförmige Färbemittel sind beispielsweise aus der US-PS 3 960 476, US-PS 6 440 175, US-PS 4 566 875, US-PS 5 261 926 und EP-OS 0 148 681 bekannt. Ebenfalls ist aus der DE-OS 102 60 880 und der DE-OS 102 30 414 die Verwendung von Formkörpern (z.B. Tabletten) sowie aus der DE-OS 195 48 291 und der DE-OS 42 33 874 die Verwendung von Granulaten bekannt.

Die genannten Färbemittel nach dem Stand der Technik sind üblicherweise gebrauchsfertig nuanciert. Dies hat den Nachteil, dass einerseits eine Vielzahl von Nuancen vorrätig gehalten werden müssen, was unter anderem zu hohen Lagerkosten führt, und andererseits die Erstellung von individuellen oder besonderen Nuancen nur schwer möglich ist.

Überraschenderweise wurde nunmehr gefunden, dass dieses Problem durch die Verwendung eines speziellen Mehrkomponenten-Kits und/oder eines speziellen Verfahrens gelöst werden kann.

Da der erfindungsgemäße Mehrkomponenten-Kit die oder den Farbstoff(e) und/oder die oxidierenden Mittel in einer Granulatform enthält, die farblich nach dem Farbkreis erstellt wurden und aufgebaut sind, kann der geschulte Fachmann unter Berücksichtigung bestimmter reaktionskinetischer Farbstoffentwicklungen aus einer überschaubaren Anzahl von Farbstoffvormischungen eine jeweils auf die Wünsche des Kunden abgestimmte individuelle Farbnuance erstellen, die zudem eine gegenüber handelsüblichen Färbemitteln intensivere Färbung ergibt.

Gegenstand der vorliegenden Erfindung ist daher ein Mehrkomponenten-Kit zum Färben von Keratinfasern, insbesondere menschlichen Haaren, welcher **dadurch gekennzeichnet** ist, dass er aus den folgenden Komponenten besteht:
(i) einer Farbstoffträgermasse (A), welche frei ist von Farbstoffen und Farbstoffvorstufen (z.B. einer farbstofffreien Flüssigfarbmasse, Creme-Gel-Farbmasse oder Cremefarbmasse);
(ii) mehreren Zusammensetzungen (B) bestehend aus farbstoffhaltigen Pellets, welche durch homogenes Vermischen eines mindestens einen natürlichen und/oder synthetischen Farbstoff enthaltenden Ausgangsstoffes mit einem geeigneten Trägermaterial und anschließende Beschichtung mit einem geeigneten Verkapselungsmaterial erhalten werden, und mindestens ein Oxidationsfarbstoffvorprodukt und/oder mindestens einen Direktfarbstoff sowie ggfs. mindestens eine keratinaufhellende bzw. bleichende Substanz enthalten; und ggfs. (iii) einem geeigneten Oxidationsmittel (C).

Ein weiterer Gegenstand der vorliegenden Erfindung ist ein Mehrkomponenten-Kit zum Färben von Keratinfasern, insbesondere menschlichen Haaren, welcher **dadurch gekennzeichnet** ist, dass er aus den folgenden Komponenten besteht:
(i) einer Farbstoffträgermasse (A), welche frei ist von Farbstoffen und Farbstoffvorstufen (z.B. einer farbstofffreien Flüssigfarbmasse, Creme-Gel-Farbmasse oder Cremefarbmasse);
(ii) mehreren Zusammensetzungen (B) bestehend aus farbstoffhaltigen Pellets, welche durch Beschichtung eines geeigneten Trägermaterials mit einer Mischung aus mindestens einem natürlichen und/oder synthetischen Farbstoff und mindestens eines geeigneten Verkapselungsmaterials erhalten werden, und mindestens ein Oxidationsfarbstoffvorprodukt und/oder mindestens einen Direktfarbstoff sowie ggfs. mindestens eine keratinaufhellende bzw. bleichende Substanz enthalten; und ggfs. (iii) einem geeigneten Oxidationsmittel (C).

Ein weiterer Gegenstand der vorliegenden Erfindung ist die Verwendung einer Kombination aus (i) einer farbstofffreien Farbstoffträgermasse (A) und (ii) mehreren Zusammensetzungen (B) bestehend aus farbstoffhaltigen Pellets, welche durch homogenes Vermischen eines mindestens einen natürlichen und/oder synthetischen Farbstoff enthaltenden Ausgangsstoffes mit einem geeigneten Trägermaterial und anschließende Beschichtung mit einem geeigneten Verkapselungsmaterial erhalten werden, und mindestens ein Oxidationsfarbstoffvorprodukt und/oder mindestens einen Direktfarbstoff sowie ggfs. mindestens eine keratinaufhellende bzw. bleichende Substanz enthalten, sowie (iii) ggfs. einem Oxidationsmittel (C) zur individuellen Herstellung von Färbemitteln für Keratinfasern unmittelbar vor der Färbung der Keratinfasern.

Ein weiterer Gegenstand der vorliegenden Erfindung ist die Verwendung einer Kombination aus (i) einer farbstofffreien Farbstoffträgermasse (A) und (ii) mehreren Zusammensetzungen (B) bestehend aus farbstoffhaltigen Pellets, welche durch Beschichtung eines geeigneten Trägermaterials mit einer Mischung aus mindestens einem natürlichen und/oder synthetischen Farbstoff und mindestens eines geeigneten Verkapselungsmaterials erhalten werden, und mindestens ein Oxidationsfarbstoffvorprodukt und/oder mindestens einen Direktfarbstoff sowie ggfs. mindestens eine keratinaufhellende bzw. bleichende Substanz enthalten, sowie (iii) ggfs. einem Oxidationsmittel (C) zur individuellen Herstellung von Färbemitteln für Keratinfasern - insbesondere menschlichen Haaren- unmittelbar vor der Färbung der Kerratinfasem.

Weiterhin betrifft die vorliegende Erfindung ein Verfahren zum Färben oder gleichzeitigen Färben und Aufhellen von Keratinfasern - insbesondere menschlichen Haaren-, gemäß dem Wortlaut des Anspruchs 6.

Die Zusammensetzungen (A), (B) und (C) werden vorzugsweise in den folgenden Mengen eingesetzt:
Komponente (A): 10 bis 120 g , insbesondere 20 bis 80 g;
Komponente (B): 0,1 bis 20 g , insbesondere 0,5 bis 12 g;
Komponente (C): 10 bis 120 g , insbesondere 20 bis 80 g.

Das Verhältnis von (A) zu (B) beträgt in der Regel 1000:1 bis 2:1, vorzugsweise 200:1 bis 4:1 und insbesondere 100:1 bis 5:1. Das Verhältnis von (C) zu (A) beträgt in der Regel 3:1 bis 1:3, vorzugsweise 1:1.

Als geeignete Oxidationsfarbstoffvorstufen können in der Zusammensetzung (B) beispielsweise die folgenden Entwicklersubstanzen und Kupplersubstanzen und mit sich selbst kuppelnden Verbindungen eingesetzt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1 H-pyrazol, 4,5-Diamino-1-methyl-1H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Unter den vorgenannten Oxidationsfarbstoffen sind die folgenden Verbindungen, alleine oder in Kombination miteinander besonders bevorzugt: 2,5-Diamino-toluol, 2,4-Diaminophenoxyethanol, Resorcin, 2-Methylresorcin, m-Aminophenol, 4-Amino-m-kresol, 4-Amino-2-hydroxytoluol, 6-Amino-m-kresol, 2-Amino-4-hydroxyethylaminoanisol, 1-Naphthol, Hydroxyethyl-3,4-methylendioxyanilin, 2,5-Diamino-phenylethanol, N,N-Bis(2-hydroxyethyl)-p-phenylendiamin, Phenyl-methyl-pyrazolon, 1-Hydroxyethyl-4,5-diamino-pyrazol und 2-Amino-6-chlor-4-nitro-phenol oder deren Salze.

Die Gesamtmenge der in der erfindungsgemäßen Zusammensetzung (B) enthaltenen Oxidationsfarbstoffvorstufen beträgt etwa 0,1 bis 70 Gewichtsprozent, insbesondere etwa 0,5 bis 50 Gewichtsprozent.

Zur Erzielung bestimmter Farbnuancen können ferner auch übliche natürliche und/oder synthetische direktziehende Farbstoffe, beispielsweise sogenannte Pflanzenfarbstoffe wie Henna oder Indigo,Triphenylmethanfarbstoffe, aromatische Nitrofarbstoffe, Azofarbstofte, Chinonfarbstoffe, kationische oder anionische Farbstoffe, zugesetzt werden. Es ist jedoch auch möglich, dass die erfindungsgemäße Zusammensetzung (B) ausschließlich direktziehende Farbstoffe enthält.

Als geeignete synthetische Farbstoffe können beispielsweise genannt werden: Hydroxyethyl-2-nitro-p-toluidin, 2-Hydroxyethyl-pikraminsäure, 4-Nitrophenyl-aminoharnstoff, Tri(4-amino-3-methylphenyl)-carbeniumchlorid (Basic Violet 2), 1,4-Diamino-9,10-anthracendion (Disperse Violet 1), 1,4-Bis[(2-hydroxyethyl)amino]-2-nitrobenzol, 1-(2-Hydroxyethyl)-amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxy-ethyl)-amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitrodiphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure 2-[(4-Amipo-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxy-ethyl)-amino]-1-methoxy-5-nitrobenzol, 2-Amino-3-nitrophenol, 1-(2-Hydroxyethoxy)-3-methylamino-4-nitrobenzol, 2,3-(Dihydroxy-propoxy)-3-methyl-amino-4-nitrobenzol, 2-[(2-Hydroxyethyl)amino]-5-nitrophenol (HC Yellow No. 11), 3-[(2-Aminoethyl)-amino]-1-methoxy-4-nitrobenzol-hydrochlorid (HC Yellow No.9), 1-[(2-Ureidoethyl)amino]-4-nitrobenzol, 4-[(2,3-Di-hydroxypropyl)amino]-3-nitro-1-trifluormethyl-benzol (HC Yellow No. 6), 1-Chlor-2,4-bis[(2-hydroxyethyl)-amino]-5-nitrobenzol (HC Yellow No. 10), 4-[(2-Hydroxy-ethyl)amino]-3-nitro-1-methylbenzol, 1-Chlor-4-[(2-hydroxy-ethyl)amino]-3-nitrobenzol (HC Yellow No. 12), 4-[(2-Hydroxyethyl)-amino]-3-nitro-1-trifluormethyl-benzol (HC Yellow No. 13), 4-[(2-Hydroxy-ethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxy-propyl)amino]-9,10-anthrachinon, 1-[(2-Hydroxy-ethyl)amino]-4-methyl-amino-9,10-anthrachinon (CI61505, Disperse Blue No. 3), 2-[(2-Amino-ethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Amino-propyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]-phenoxazin-7-ium-chlorid (CI51175; Basic Blue No. 6), Di[4-(diethyl-amino)phenyl][4-(ethylamino)naphthyl]-carbenium-chlorid (CI42595; Basic Blue No. 7), 3,7-Di(dimethylamino)-phenothiazin-5-ium-chlorid (CI52015; Basic Blue No. 9), Di[4-(dimethyl-amino)phenyl][4-(phenylamino)naphthyl]-carbenium-chlorid (CI44045; Basic Blue No. 26), 2-[(4-(Ethyl(2-hydroxy-ethyl)amino)phenyl)azo]-6-methoxy-3-methylbenzothiazolium-methylsulfat (CI11154; Basic Blue No. 41), 8-Amino-2-brom-5-hydroxy-4-imino-6-[(3-(trimethylammonio)phenyl)-amino]-1(4H)-naphthalinon-chlorid (CI56059; Basic Blue No. 99), Bis[4-(dimethylamino)phenyl][4-(methyl-amino)phenyl]carbenium-chlorid (CI42535; Basic Violet No. 1), Tris[4-(dimethylamino)phenyl]carbenium-chlorid (CI42555; Basic Violet No. 3), 2-[3,6-(Diethylamino)-dibenzopyranium-9-yl]-benzoesäure-chlorid (CI45170; Basic Violet No. 10), Di(4-aminophenyl)-(4-amino-3-methyl-phenyl)carbenium-chlorid (CI42510; Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethyl-ammonio)-2-naphtholchlorid (CI12251; Basic Brown No. 17), 3,7-Diamino-2,8-dimethyl-5-phenyl-phenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 2-Hydroxy-1-[(2-methoxy-phenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Bis[4-(diethylamino)phenyl]phenylcarbenium-hydrogensulfat(1:1) (CI42040; Basic Green No. 1), 1-[Di(2-hydroxyethyl)-amino]-3-methyl-4-[(4-nitro-phenyl)azo]-benzol (CI11210, Disperse Red No. 17), 4-[(4-Aminophenyl)-azo]-1-[di(2-hydroxyethyl)amino]-3-methyl-benzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(indan-1,3-dion-2-yl)chinolin-x,x-sulfonsäure (Gemisch aus Mono- und Disulfonsäure) (CI47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)amino]-2-phenylamino-benzolsulfonsäure-natriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2, 4-naphthalindisulfonsäure-trinatriumsalz (CI16255; Ponceau 4R; Acid Red No. 18), 3-Hydroxy-4-[(4-sulfonaphth-1-yl)azo]-2,7-naphthalin-disulfonsäuretrinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäuredinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100, Acid Red No. 52), 8-[(4-(Phenylazo)-phenyl)azo]-7-naphthol-1,3-disulfonsäure-dinatriumsalz (CI27290; Acid Red No. 73), 2',4',5',7'-Tetrabrom-3',6'-dihydroxyspiro-[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1 (3H),9'(9H)-xanthen]-3-on-dinatriumsalz (CI45425; Acid Red No. 95), (2-Sulfophenyl)di[4-(ethyl((4-sulfophenyl)methyl)amino)phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62),2-(1,3-Dihydro-3-oxo-5-sulfo-2H-indol-2-yliden)-2,3-dihydro-3-oxo-1 H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalindisulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (Cl14700; Food Red No. 1; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1) und 3-Hydroxy-4-(3-methyl-5-oxo-1-phenyl-4,5-dihydro-1 H-pyrazol-4-ylazo)-naphthalin-1-sulfonsäure-natriumsalz, Chrom-Komplex (Acid Red No. 195), 3',3",4,5,5',5",6,7-Octabromphenolsulfonphtalein (Tetrabromphenolblue), 1-((4-Amino-3,5-dimethylphenyl)-(2,6-dichlorphenyl)methylen)-3,5-dimethyl-4-imino-2,5-Cyclohexadien-verb mit Phosphorsäure(1:1) (Basic Blue 77), 2',4',5',7'-tetrabrom-4,5,6,7-tetrachlor-3',6'-dihydroxy-Spiro[isobenzofuran-1 (3H),9'[9H]xanthen]-3-on-dinatriumsalz (Acid Red 92), N,N-Di(2-hydroxyethyl)-3-methyl-4-((4-nitrophenyl)azo)-anilin (Disperse Red 17), 2,4-Dinitro-1-naphthol-7-sulfonsäure-dinatriumsalz (Acid Yellow 1), 4-((2-Hydroxynaphthalin-1-yl)azo)-benzolsulfonsäure-natriumsalz (Acid Orange 7), 2-((4-(Ethyl(2-hydroxyethyl)amino)-2-methylphenyl)azo)-5-nitro-1,3-thiazol (Disperse Blue 106), 2,4-Dinitro-1-naphtol, 2-[(4-Aminophenyl)-azo]-1,3-dimethyl-1H-imidazo-3-lium-chlorid, 1-Methyl-4-((methylphenylhydrazono)methyl)-pyridiniummethosulfat, 2-[[4-(Dimethylamino)phenyl]-azo]-1,3-dimethyl imidazolium-chlorid, 2-((4-((4-Methoxyphenyl)amino)-phenyl)azo)-1,3-dimethyl-1H-Imidazol-3-ium-chlorid und 1,3-Dimethyl-2-((4-((phenylmethyl)amino) phenyl)azo)-1H-Imidazol-3-ium-chlorid, alleine oder in Kombination miteinander,

Unter den vorgenannten direktziehenden Farbstoffen sind die folgenden Verbindungen -alleine oder in Kombination miteinander- besonders bevorzugt: Hydroxyethyl-2-nitro-p-toluidin, 2-Hydroxyethyl-pikraminsäure, 4-Nitrophenyl-aminoharnstoff, Tri(4-amino-3-methylphenyl)-carbeniumchlorid (Basic Violet 2), 1,4-Diamino-9,10-anthracendion (Disperse Violet 1), 1-(2-Hydroxyethyl)-amino-2-nitro-4-[di(2-hydroxyethyl)-amino]-benzol (HC Blue No. 2), 4-[Ethyl-(2-hydroxyethyl)amino]-1-[(2-hydroxyethyl)-amino]-2-nitro-benzol-hydrochlorid (HC Blue No. 12), 1-Amino-4-[di(2-hydroxyethyl)amino]-2-nitrobenzol-hydrochlorid (HC Red No. 13), 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-Chlor-6-ethylamino-4-nitrophenol, 2-Amino-6-chlor-4-nitrophenol, 4-[(2-Hydroxyethyl)-amino]-3-nitro-1-trifluormethyl-benzol (HC Yellow No. 13), 8-Amino-2-brom-5-hydroxy-4-imino-6-[(3-(trimethylammonio)phenyl)-amino]-1(4H)-naphthalinon-chlorid (CI56059; Basic Blue No. 99), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 2-Hydroxy-1-[(2-methoxy-phenyl)azo]-7-(trimethylammonio)-naphthalin-chlorid (CI12245; Basic Red No. 76), 3-Methyl-1-phenyl-4-[(3-(trimethylammonio)-phenyl)azo]-pyrazol-5-on-chlorid (CI12719; Basic Yellow No. 57) und 2,6-Diamino-3-[(pyridin-3-yl)azo]-pyridin sowie deren Salze.

Die Gesamtmenge der direktziehenden Farbstoffe beträgt in der erfindungsgemäßen Zusammensetzung (B) etwa 0,1 bis 90 Gewichtsprozent, vorzugsweise 1 bis 70 Gewichtsprozent.

Weitere für die Verwendung in Haarfärbemitteln bekannte und übliche Farbstoffe sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben, auf die heirmit ausdrücklich Bezug genommen wird. Die Zubereitungsform der erfindungsgemäßen Farbstoffträgermasse (A) kann beispielsweise eine Lösung, insbesondere eine wässrige oder wässrig-alkoholische Lösung sein. Die besonders bevorzugten Zubereitungsformen sind jedoch eine Creme, ein Gel oder eine Emulsion.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann die Farbträgermasse (A) schwach sauer, neutral oder alkalisch reagieren. Insbesondere weist sie einen pH-Wert von 6,5 bis 11,5 auf. Die basische Einstellung erfolgt hierbei vorzugsweise mit Ammoniak, wobei jedoch auch organische Amine, zum Beispiel Monoethanolamin oder Triethanolamin, oder anorganische Basen, beispielsweise Natriumhydroxid oder Kaliumhydroxid Verwendung finden können. Für eine PH Einstellung im sauren Bereich kommen anorganische oder organische Säuren, zum Beispiel Phosphorsäure, Essigsäure, Zitronensäure oder Weinsäure, in Betracht.

In der Farbträgermasse (A) können beispielsweise auch Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 0,5 Gewichtsprozent enthalten sein. Parfümöle können in der erfindungsgemäßen Farbträgermasse in einer Menge von bis zu etwa 1 Gewichtsprozent enthalten sein. Weiterhin kann die erfindungsgemäße Komponente (A) für derartige Mittel übliche Hilfs- und Zusatzstoffe, wie zum Beispiel Verdickungsmittel, beispielsweise Homopolymere der Acrylsäure, Pflanzen-Gums, Algenpolyasaccharide, amphiphile Assoziatiwerdicker, desweiteren Konservierungsstoffe; Komplexbildner; Netzmittel oder Emulgatoren aus den Klassen der anionischen, kationischen, amphoteren oder nichtionogenen oberflächenaktiven Substanzen; Alkalisierungsmittel (z.B. Ammoniumsalze und/oder Aminosäuren, wie z.B. Glycin und Alanin); sowie Pflegestoffe, wie kationische Polymere oder Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent.

Die erfindungsgemäße Komponente (C) enthält einen oder mehrere bekannte chemische Oxidationsmittel, beispielsweise Wasserstoffperoxid oder dessen Salze oder Addukte sowie Persulfate wie Natriumpersulfat, Kaliumpersulfat oder Ammoniumpersulfat.

Besonders bevorzugte granulatförmige Zusammensetzungen der Komponente (B) sind farbstoffhaltige Pellets, welche (a) durch homogenes Vermischen eines mindestens einen natürlichen und/oder synthetischen Farbstoff enthaltenden Ausgangsstoffes mit einem geeigneten Trägermaterial und anschließende Beschichtung mit einem geeigneten Verkapselungsmaterial oder (b) durch Beschichtung eines geeigneten Trägermaterials mit einer Mischung aus mindestens einem natürlichen und/oder synthetischen Farbstoff und mindestens eines geeigneten Verkapselungsmaterials erhalten werden.

Diese farbstoffhaltigen Pellets werden entweder nach dem in der *Anlage 1* schematisch dargestellten Verfahren mittels Extrudertechnologie [Pellets gemäß (a)] oder nach dem in der *Anlage 2* schematisch dargestellten Top-Spray-Verfahren [Pellets gemäß (a) oder (b)] hergestellt. Beispielsweise kann das Verfahren für einen 1 Kilogramm-Ansatz wie folgt durchgeführt werden, wobei bei größeren Ansätzen der Sprühluftdruck und die Sprührate entsprechend angepasst werden müssen:

### 1. Herstellung mittels Extrudertechnologie:

Hierzu wird in einem Vertikalgranulierer (Rotordrehzahl = 50 bis 200 U/min, vorzugsweise etwa 150 U/min; Zerhackerdrehzahl = 750 bis 1250 U/min, vorzugsweise etwa 1000 U/min) bei Raumtemperatur (15 bis 35 °C) durch Trockenmischen und anschließendes Nassmischen der Farbstoffmasse mit den Trägermaterialien und ggfs. Antioxidantien und weiteren Hilfsstoffen eine Grundmasse hergestellt. Anschließend wird diese Grundmasse in einem Extruder (Drehzahl = 15 bis 50 U/min, vorzugsweise etwa 25 bis 30 U/min; Lochgröße der Siebe = etwa 0,01 bis 5 mm; vorzugsweise 0,1 bis 3 mm und insbesondere 0,6 bis 1 mm) extrudiert und das so erhaltene Granulat in einem Pelletizer (Drehzahl = 400 bis 800 U/min; vorzugsweise etwa 500 bis 600 U/min) verrundet. Das Granulat wird sodann bei einer Produkttemperatur von 20 bis 60 °C (vorzugsweise 30 bis 55 °C) getrocknet (Zulufttemperatur vorzugsweise etwa 70 bis 80 °C) und anschließend (ggfs. nach vorherigem Erwärmen auf 40-50 °C) mit Hilfe des Wirbelschichtverfahrens (Sprührate vorzugsweise gleich etwa 5 bis 20 g/min; Sprühluftdruck vorzugsweise gleich etwa 1,5 bis 2,5 bar) gecoatet, wobei die Menge an verwendetem Verkapselungsmaterial (bezogen auf die Menge des zu coatenden Granulats) 0,5 bis 50 Gewichtsprozent, vorzugsweise 1 bis 20 Gewichtsprozent und insbesondere 2 bis 15 Gewichtsprozent, beträgt. Abschließend wird das Produkt getrocknet (Produkttemperatur max. etwa 60 °C).

### 2. Herstellung mittels Top-Spray-Verfahren:

(a) Hierzu wird in einem Wirbelschichtgranulierer/Coater bei Raumtemperatur (15 bis 35 °C) die Farbstoffmasse mit den Trägermaterialien und ggfs. Antioxidantien und weiteren Hilfsstoffen vermischt. Anschließend wird die so erhaltene Grundmasse temperiert (Produkttemperatur max. etwa 55 °C), sodann granuliert (Sprührate vorzugsweise gleich etwa 6 bis 20 g/min; Sprühluftdruck vorzugsweise gleich etwa 0,25 bis 2,5 bar), wobei die Menge an verwendetem Verkapselungsmaterial (bezogen auf die Menge des zu coatenden Granulats) 0,5 bis 50 Gewichtsprozent, vorzugsweise 1 bis 20 Gewichtsprozent und insbesondere 2 bis 10 Gewichtsprozent, beträgt; und gegebenenfalls anschließend gecoatet. Falls erforderlich wird das Produkt abschließend getrocknet (Produkttemperatur max. etwa 60 °C).
(b) Hierzu werden in einem Wirbelschichtgranulierer/Coater bei Raumtemperatur (15 bis 35 °C) die Trägermaterialien und ggfs. Antioxidantien und weiteren Hilfsstoffe miteinander vermischt. Anschließend wird die so erhaltene Grundmasse temperiert (Produkttemperatur max. etwa 55 °C), sodann granuliert und anschließend mit einer Lösung/Dispersion der Farbstoffe und ggfs. Antioxidantien und weiterer Hilfsstoffe in einem geeigneten Verkapselungsmaterial gecoatet (Sprührate vorzugsweise gleich etwa 6 bis 20 g/min; Sprühluftdruck vorzugsweise gleich etwa 0,25 bis 2,5 bar) gecoatet, wobei die Menge an verwendetem Verkapselungsmaterial (bezogen auf die Menge des zu coatenden Granulats) 0,5 bis 50 Gewichtsprozent, vorzugsweise 1 bis 20 Gewichtsprozent und insbesondere 2 bis 10 Gewichtsprozent, beträgt. Falls erforderlich wird das Produkt abschließend getrocknet (Produkttemperatur max. etwa 60 °C).

Geeignete Trägermaterialien für die farbstoffhaltigen Pellets sind pulvrige, mikrokristalline Substanzen die die Farbstoffe in einen physikalischen Zustand versetzen, der es erlaubt, das Verfahren zum Coaten der Pellets mit geeigneten Verkapselungsmaterialien durchzuführen.
Geeignete Trägermaterialien sind insbesondere Gummi Arabicum, Dextrose, Polyvinylpyrrolidon, Oligosaccharide, mikrokristalline CelluloseDerivate, wie zum Beispiel Hydroxypropylmethylcellulose, Methylcellulose, Hydroxyethyl-cellulose, Hydroxypropylcellulose, Nonoxynol-Hydroxyethylcellulose und Cetyl-Hydroxyethylcellulose oder physikalisch beziehungsweise chemisch modifizierte Stärken oder Stärkederivate, wie zum Beispiel Stärkeester (bveispielsweise acetylierte Stärken), Stärkeether (beispielsweise hydroxyalkylierte Stärken), Dialdehydstärken, Dicarboxylstärken, Distärkephosphate, Hydroxyalkylstärkephosphate oder Hydroxyalkylstärken, wobei die Alkylgruppen vorzugsweise 1 bis 4, besonders bevorzugt 2 bis 3 C-Atome enthalten. Geeignet sind auch quervernetzte Stärkeether, wie zum Beispiel solche mit den INCl-Bezeichnungen Dimethylimidazolidone Rice beziehungsweise Corn Starch oder hydrophob modifizierte Stärken (beispielsweise solche mit der INCl-Bezeichnung Aluminium Starch Octensuccinate). Die Stärke kann sowohl thermisch als auch hydrolytisch oder enzymatisch modifiziert worden sein, wobei die Ausgangsstärke aus den bekannten Quellen, beispielsweise Mais, Kartoffeln, Süsskartoffeln, Erbsen, Bananen, Hafer, Weizen, Gerste, Reis, Sago, Tapioca, Pfeilwurz, Amarant, Kanna, Sorghum, usw., gewonnen werden kann. Besonders bevorzugte Stärkederivate sind nichtionische Stärkederivate, insbesondere mit Alkylenoxiden wie Ethylenoxid, Propylenoxid oder Butylenoxid, Acetanhydrid oder Butylketendimer, und insbesondere Propylenoxid, modifizierte nichtionische Stärkederivate. Weitere geeignete Trägermaterialien sind synthetisches Calciumsilicat, Kieselgur und Siliziumdioxid.

Geeignete Verkapselungsmaterialien für die erfindungsgemäßen Pellets sind wasserlösliche oder wasserdispergierbare, filmbildende Substanzen, die in der Lage sind, aus Lösungen oder Dispersionen, sich durch Sprühtrocknung derart auf den Pellets einheitliche Filme abzuscheiden, dass von einer Ummantelung (Coating) gesprochen werden kann.
Geeignete Verkapselungsmaterialien sind Gummi Arabicum, CelluloseDerivate (beispielsweise Methylcellulosen), Polyethylen-Dispersionen, Polyacrylsäuren, Polyvinylalkohole, Polycarbonate, Polyvinylpyrrolidon, Polyester und Polyamide oder natürliche Filmbildner wie zum Beispiel Chitosan, Schellack, Oligosaccharide oder auch chinesisches Balsamharz (Kolophonium).

Die eingesetzten Pellets weisen vorzugsweise einen Teilchendurchmesser von 100 µm bis 1000 µm, insbesondere einen Teilchendurchmesser von 120 µm bis 1500 µm, auf.

Für die Anwendung vermischt man unmittelbar vor dem Gebrauch die Komponenten (A) und (B) und bei der oxidativen Färbung bzw. der gleichzeitigen Aufhellung und Färbung von Haaren zusätzlich Komponente (C) miteinander und trägt eine für die Haarfärbebehandlung ausreichende Menge des so hergestellten individuell abgestimmten Färbemittels, je nach Haarfülle, im allgemeinen etwa 60 bis 200 Gramm, auf das haar auf.

Falls eine Aufhellung gewünscht wird, können in der Komponente (B) geeignete keratinaufhellende Stoffe (insbesondere Persulfate und Mischungen aus Persulfaten und Wasserstoffperoxid) enthalten.

Das erfindungsgemäße Mittel (C) kann mindestens eine Quelle für ein Oxidationsmittel enthalten. Bevorzugte Oxidationsmittel sind wasserlösliche peroxidhaltige Oxidationsmittel. "Wasserlöslich" bedeutet heirbei, dass unter Standardbedingungen mindestens 0,1 g, vorzugsweise 1 g und insbesondere 10 g des Oxidationsmittels in 1 Liter entmineralisiertem Wasser gelöst werden können. Die Oxidationsmittel sind für die anfängliche Solubilisierung und Entfärbung des Melanins (Bleichen) und die Oxidation der Oxidationsfarbstoffvorstufen (oxidative Färbung) im Haarschaft nützlich.
Jedes bekannte wasserlösliche Oxidationsmittel kann im Rahmen der vorliegenden Erfindung verwendet werden, wobei anorganische Peroxide, welche in einer wässrigen Lösung Wasserstoffperoxid bilden, bevorzugt sind. Wasserlösliche peroxidhaltige Oxidationsmittel sind aus dem Stand der Technik hinreichend bekannt und beinhalten Wasserstoffperoxid, anorganische Metallperoxide wie zum Beispiel Natriumperjodat oder Natriumperoxid, organische Peroxide wie zum Beispiel Harnstoffperoxid oder Melaminperoxid und anorganische Persalz-Bleichmittel, wie zum Beispiel Alkalimetallsalze der Perborate, Percarbonate, Perphosphate, Persilikate, Persulfate und ähnliche. Diese anorganischen Persalze können auch als Monohydrate, Tetrahydrate, usw. enthalten sein. Alkyl- und Arylperoxide und/oder Peroxidasen können ebenfalls verwendet werden. Wenn gewünscht können auch Mischungen von 2 oder mehreren dieser Oxidationsmittel eingesetzt werden. Die Oxidationsmittel können in Form einer wässrigen Lösung oder eines Pulvers, das vor der Anwendung gelöst wird, vorliegen. Bevorzugte Oxidationsmittel gemäß der vorliegenden Erfindung sind Wasserstoffperoxid, Percarbonate, Persulfate und Kombinationen dieser Verbindungen.
Gemäß der vorliegenden Erfindung enthalten die Mittel etwa 0,1 bis etwa 15 Gewichtsprozent, vorzugsweise etwa 1 bis 10 Gewichtsprozent, und insbesondere etwa 2 bis etwa 7 Gewichtsprozent des Oxidationsmittels. Ein weiteres bevorzugtes Oxidationsmittel ist eine Quelle für Peroxymonocarbonationen, die vorzugsweise in-situ aus einer Wasserstoffperoxid-Quelle und einer Hydrogencarbonat-Quelle gebildet wird. Derartige Oxidationsmittel sind besonders effektiv bei einem pH-Wert von kleiner/gleich 9,5, wobei ein pH-Wert von 7,5 bis 9,5 und insbesondere etwa 9 bevorzugt ist. Weiterhin ist dieses System besonders effektiv in Kombination mit einer Quelle für Ammoniak oder Ammoniumionen. Es hat sich gezeigt, dass Oxidationsmittel die gewünschten Haarfärbeergebnisse positv beeinflussen können, bei gleichzeitiger deutlicher Senkung des Geruches, der Haut- und Kopfhautirritationen sowie der der Haarschädigung.
Jede Quelle dieser Ionen kann verwendet werden, wobei als geeignete Quellen beispielsweise Natrium-, Kalium-, Guanidin-, Arginin-, Lithium-, Calcium-, Magnesium-, Barium- oder Ammoniumsalze der Carbonate, Carbamate und Hydrogencarbonate sowie deren Mischungen, beispielsweise Natriumcarbonat, Natriumhydrogencarbonat, Kaliumcarbonat, Kaliumhydrogencarbonat, Guanidincarbonat, Guanidinhydrogencarbonat, Lithiumcarbonat, Calciumcarbonat, Magnesiumcarbonat, Bariumcarbonat, Ammoniumcarbonat, Ammoniumhydrogencarbonat und deren Mischungen, genannt werden können. Percarbonatsalze können gleichzeitig als Carbonationen-Quelle und als Oxidationsmittel dienen. Bevorzugte Quellen für Carbonate, Carbamate und Hydrogencarbonate sind Natriumhydrogencarbonat, Kaliumhydrogencarbonat und Ammonium carbamat sowie deren Mischungen.
Gemäß der vorliegenden Erfindung enthalten die Mittel etwa 0,1 bis etwa 15 Gewichtsprozent, vorzugsweise etwa 1 bis etwa 10 Gewichtsprozent und insbesondere etwa 1 bis etwa 8 Gewichtsprozent eines Hydrogencarbonations und etwa 0,1 bis 10 Gewichtsprozent, vorzugsweise etwa 1 bis 7 Gewichtsprozent und insbsondere etwa 2 bis etwa 6 Gewichtsprozent einer Wasserstoffperoxide-Quelle.

Gemäß der vorliegenden Erfindung können die Mittel gegebenenfalls weiterhin mindestens eine Quelle für ein Alkalisierungsmittel, vorzugsweise eine Quelle für Ammoniak oder Ammoniumionen, enthalten. Als Alkalisierungsmittel kann jede bekannte Verbindung, beispielsweise Alkanolamide wie zum Beispiel Monoethanolamin, Diethanolamin, Triethanolamin, Monopropanolamin, Dipropanolamin, Tripropanolamin, 2-Amino-2-methyl-1, 3-propandiol, 2-Amino-2-methyl-1-propanol oder 2-Amino-2-hydroxymethyl-1,3-propandiol, und Guanidiumsalze, verwendet werden. Besonders bevorzugte Alkalisierungsmittel sind solche Mittel, welche eine Quelle für Ammoniumionen aufweisen, wobei jede Quelle für Ammoniumionen geeignet ist. Bevorzugte Quellen für Ammoniumionen sind Ammoniumchlorid, Ammoniumsulfat, Ammoniumnitrat, Ammoniumphosphat, Ammoniumacetat, Ammoniumcarbonat, Ammoniumhydrogencarbonat, Ammoniumcarbamat, Ammoniumhydroxid, Percarbonatsalze, Ammoniak und deren Mischungen. Besonders bevorzugt sind hierbei Ammoniumcarbonat, Ammoniumcarbamat, Ammoniumhydrogencarbonat, Ammoniak und deren Mischungen. Die Mittel gemäß der vorliegenden Erfindung können etwa 0,1 bis etwa 10 Gewichtsprozent, vorzugsweise etwa 0,5 bis etwa 5 Gewichtsprozent, und insbesondere etwa 1 bis etwa 3 Gewichtsprozent eines Alkalisierungsmittels, vorzugsweise Ammoniumionen, enthalten. Gemäß der vorliegenden Erfindung können die Mittel weiterhin eine Quelle für einen Radikalfänger enthalten. Der Begriff "Radikalfänger" beschreibt im vorliegenden Fall eine Verbindung, die mit einem reaktiven Radikal, vorzugsweise Carbonatradikalen, reagiert, um diese reaktiven Radikale mittels einer Reihe von schnellen Reaktionen in eine weniger reaktive Verbindung umzuformen.
Geeignete Radikalfänger sind Verbindungen der allgemeinen Formel (I)

R¹-Y-C(H)(R³)-R⁴-(C(H)(R⁵)-Y-R⁶)ₙ (I)

mit Y gleich NR², O, oder S, vorzugsweise NR², n gleich 0 bis 2, und worin R⁴ ein monovalenter oder divalenter Rest aus der Gruppe bestehend aus:
(a) substitutierten oder unsubstitutierten, geradkettigen oder verzweigten Alkyl- oder einfach- oder mehrfach-ungesättigten Alkyl- oder Heteroalkylgruppen und aliphatischen, heteroaliphatischen oder heteroolefinischen Systemen, (b) substitutierten oder unsubstitutierten, mono- oder polyzyklischen aliphatischen, aromatischen oder heterozyklischen Systemen, oder (c) substitutierten oder unsubstitutierten, mono-, poly- oder perfluorierten Alkylsystemen ist; wobei die Systeme (a), (b) und (c) 1 to 12 Kohlenstoffatome und 0 bis 5 Heteroatome (O, S, N, P, Si) aufweisen; und worin R⁴ gemeinsam mit R³ oder R⁵ einen 5-, 6- oder 7-gliedrigen Ring bilden; und worin R¹, R², R³, R⁵ und R⁶ monovalent sind und unabhängig voneinander eine der vorstehend beschriebenen Gruppen (a), (b) oder (c) oder Wasserstoff darstellen.
   R⁴ ist vorzugsweise gleich (a) einer substituierten oder unsubstituierten, geradkettigen oder verzweigten Alkyl- oder Heteroalkylgruppe, oder einem aliphatischen, heteroaliphatischen oder heteroolefinischen System,
(b) einem substitutierten oder unsubstitutierten, mono- oder polyzyklischen aliphatischen, aromatischen oder heterozyklischen System, oder (c) einem substitutierten oder unsubstitutierten, mono-, poly- oder perfluorierten Alkylsystem, wobei R⁴ gleich einer substitutierten oder unsubstitutierten, geradkettigen oder verzweigten Alkyl- oder Heteroalkylgruppe, oder einem aliphatischen oder heteroaliphatischen System, (b) einem substitutierten oder unsubstitutierten aromatischen oder heterozyklischen System, (c) einem substitutierten oder unsubstitutierten, mono-, poly- oder perfluorierten Alkylsystem und insbesondere einer substitutierten oder unsubstitutierten, geradkettigen oder verzweigten Alkyl- oder Heteroalkylgruppe, besonders bevorzugt ist. Vorzugsweise weisen die vorstehend beschriebenen R⁴-Gruppen (a), (b), and (c), 1 bis 8 Kohlenstoffatome auf, wobei 1 bis 6 Kohlenstoffatome und insbesondere 1 to 4 Kohlenstoffatome besonders bevorzugt sind, sowie 0 bis 3 Heteroatome, vorzugsweise 0 bis 2 Heteroatome und insbesondere 1 Heteroatom. Sofern das System Heteroatome aufweist, enthält es vorzugsweise 1 Heteroatom. Als Heteroatom sind insbesondere zu nennen: O, S und N, wobei O und N, und insbesondere O bevorzugt sind. R¹, R², R³, R⁵ und R⁶ stellen unabhängig voneinander Wasserstoff oder eines der für R⁴ genannten Systeme dar.
   Alternativ können R¹, R², R³, R⁴, R⁵, and R⁶ auch substituiert sein; wobei der Substituent vorzugsweise ausgewählt wird aus: (a) C-verknüpften monovalenten Substituenten aus der Gruppe der (i) substitutierten oder unsubstituierten, geradkettigten oder verzweigten Alkylreste, einfach- oder mehrfach ungesättigten Alkyl-, Heteroalkyl-, aliphatischen, heteroaliphatischen oder heteroolefinischen Systeme, (ii) substitutierten oder unsubstituierten, mono- oder polyzyklischen aliphatischen, aromatischen oder heterozyklischen Systeme, oder (iii) substitutierten oder unsubstituierten, Mono-, Poly- oder Perfluoroalkyl-Systeme; wobei die vorgenannten Systeme (i), (ii) und (iii) 1 bis 10 Kohlenstoffatome und 0 bis 5 Heteroatome (O, S, N, P, Si) enthalten; (b) S- verknüpften monovalenten Substituenten aus der Gruppe SA¹, SCN, SO₂A¹, SO₃A¹, SSA¹, SOA¹, SO₂NA¹A², SNA¹A² und SONA¹A², (c) O- verknüpften monovalenten Substituenten aus der Gruppe OA¹, OCN und ONA¹A²; (d) N- verknüpften monovalenten Substituenten aus der Gruppe NA¹A², (NA¹A²A³)⁺, NC, NA¹OA², NA¹SA², NCO, NCS, NO₂ N=NA¹, N=NOA¹, NA¹CN, NA¹NA²A³; (e) den monovalent Substituenten COOA¹, CON₃, CONA¹₂, CONA¹COA², C(=NA¹)NA¹A², CHO, CHS, CN, NC und X; und (f) Fluoroalkyl monovalenten Substituenten aus der Gruppe der Mono-, Poly-, oder Perfluoroalkyl-Systeme mit 1 bis 12 Kohlenstoffatomen und 0 bis 4 Heteroatomen.
   In den vorgenannten Gruppen (b) bis (e) sind A¹, A² und A³ monovalent und stellen unabhängig voneinander (1) H, (2) substitutierte oder unsubstituierte, geradkettigte oder verzweigte Alkylgruppen, einfach- oder mehrfach-ungesättigte Alkylgruppen, Heteroalkylgruppen, aliphatische, heteroaliphatische oder heteroolefinische Systeme, (3) substitutierte oder unsubstituierte, mono- oder polyzyklische aliphatische, aromatische oder heterozyklische Systeme, oder (4) substitutierte oder unsubstituierte, Mono-, Poly-, oder Perfluoroalkyl-Systeme dar; wobei die vorgenannten Systems (2), (3) und (4) 1 bis 10 Kohlenstoffatome und 0 bis 5 Heteroatome (O, S, N, P, Si) aufweisen; und worin X ein Halogenatom (F, Cl, Br, J) ist.
   Bevorzugte Substituenten haben einen Hammett Sigma Para (σₚ) Wert von -0,65 bis +0,75, vorzugsweise -0,4 bis +0.5. Hammett Sigma Werte werden in der Literatur "Advanced Organic Chemistry - Reactions, Mechanisms and Structure" (Jerry March, 5th ed. (2001) at pages 368-375) beschrieben, auf die hiermit ausdrücklich Bezug genommen wird.

Ebenfalls geeignete Radikalfänger sind Verbindungen der allgemeinen Formel (II): 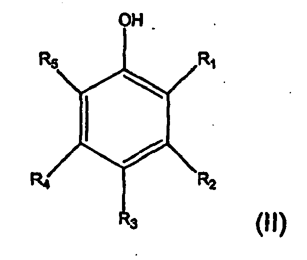 mit R₁, R₂, R₃, R₄, und R₅ unabhängig voneinander gleich H, COO-⁻M⁺, Cl, Br, SO₃⁻M⁺, NO₂, OCH₃, OH oder einer C¹ bis C¹⁰ primären oder sekundären Alkylgruppe und M entweder gleich H oder einem AlkalimetallAtom. Vorzugsweise haben die vorgenannten Radikalfänger einen pKa-Wert von 8,5 um eine Protonierung der Hydroxygruppe sicherzustellen. Weitere geeignete Radikalfänger sind solche, welche aus der Gruppe (III), bestehend aus Benzylamin, Imidazol, di-tert-Butylhydroxytoluol, Hydrochinon, Guanin, Pyrazin, Piperidin, Morpholin, Methylmorpholin, 2-Methyoxyethylamin und deren Mischungen, ausgewählt sind.
Gemäß der vorliegenden Erfindung bevorzugte Radikalfänger sind Alkanolamine, Aminozucker, Aminosäuren, Aminosäureester und deren Mischungen. Besonders bevorzugt sind: Monoethanolamin, 3-Amino-1-propanol, 4-Amino-1-butanol, 5-Amino-1-pentanol, 1-Amino-2-propanol, 1-Amino-2-butanol, 1-Amino-2-pentanol, 1-Amino-3-pentanol, 1-Amino-4-pentanol, 3-Amino-2-methylpropan-1-ol, 1-Amino-2-methylpropan-2-ol, 3-Aminopropan-1,2-diol, Glucosamin, N-Acetylglucosamin, Glycin, Arginin, Lysin, Prolin, Glutamin, Histidin, Sarcosin, Serin, Glutaminsäure, Tryptophan und deren Mischungen, sowie deren Salze, wie zum Beispiel Kalium-, Natrium und Ammoniumsalze oder deren Mischungen. Besonders bevorzugte Verbindungen sind Glycin, Sarcosin, Lysin, Serin, 2-Methoxyethylamin, Glucosamin, Glutaminsäure, Morpholin, Piperdin, Ethylamin, 3-Amino-1-propanol und deren Mischungen. Die Radikalfänger gemäß der vorliegenden Erfindung haben vorzugsweise ein Molekulargewicht unter etwa 500, vorzugsweise weniger als etwa 300, insbesondere weniger als etwa 250, um die Penetration des Radikalfängers in die Haarfaser zu erleichtern. Die Mittel gemäß der vorliegenden Erfindung enthalten -wenn sie einen Radikalfänger enthalten- vorzugsweise etwa 0,1 bis etwa 10 Gewichtsprozent, insbesondere 1 bis 7 Gewichtsprozent, des Radikalfängers. Der Radikalfänger wird vorzugweise so ausgewählt, dass er nicht vom gleichen Verbindungstyp ist wie das Alkalisierungsmittel. Gemäß einer Ausführungsform der vorliegenden Erfindung kann der Radikalfänger auch vor der Anwendung in dem Haarfärbemittel in-situ gebildet werden.

Man läßt das Gemisch sodann bei 15 bis 50 Grad Celsius etwa 10 bis 45 Minuten lang, vorzugsweise 15 bis 30 Minuten lang, auf das Haar einwirken, spült sodann das Haar mit Wasser aus und trocknet es. Im Anschluß an diese Spülung kann das Haar zusätzlich mit einem Shampoo gewaschen und gegebenenfalls mit einer schwachen organischen Säure, wie zum Beispiel Zitronensäure oder Weinsäure, nachgespült werden. Abschließend wird das Haar getrocknet.

Während Färbemittel nach dem Stand der Technik industriell gefertigt sind und bereits gebrauchsfertig nuanciert sind, ermöglicht das erfindungsgemäße Färbeverfahren dem Fachmann eine beliebige, auf die jeweils gewünschte Farbnuance abgestimmte Kombination der in Pulver- oder Granulatform vorliegenden Farbgrundtöne orange, rot, blau, gelb, usw.) und ggfs. keratinaufhellenden Verbindungen. Der Friseur muß somit nicht mehr eine Vielzahl von einzelnen Farbnuancen vorrätig halten, sondern kann die gewünschten Farbnuancen mit wenigen Komponenten (etwa 7-10 Farbstoffvormsichungen (Grundtöne) in Pulver- oder Granulatform; nur einer einzigen Grundmasse und zusätzlich einem Peroxidträger) herstellen. Neben der Verringerung des Lageraufwandes für die Färbe- und Blondiermittel ermöglicht das erfindungsgemäße Verfahren auch eine wesentlich größere Nuancenvielfalt und Kreativität des Fachmanns.

Die nachfolgenden Beispiele sollen den Erfindungsgegenstand näher erläutern ohne diesen jedoch einzuschränken.

### Beispiele

### Beispiel 1: Herstellung von Farbstoffpellets im Top-Spray-Verfahren

In einem Glatt-Wirbelschichtgranulator und Coater wird die folgende Mischung A bei einer Zulufttemperatur von 90 °C und einer Luftmenge von 18 m³/h auf eine Produkttemperatur von 34 °C erwärmt.

### Farbstoffpelletvormischung

| | |
|---|---|
| 381,2 g | 4-(2Hydroxyethylamino)-3-nitrophenol |
| 101,0 g | 2-((2-Hydroxyethyl)amino)-4,6-dinitro-phenol |
| 100,0 g | Corn Starch (Maisstärke) |

Anschließend wird eine 20 %ige wässrige Polyvinylpyrrolidon-Lösung ("Sprühlösung") mit einer Anfangs-Sprührate von 8 g/min und einem Sprühluftdruck von 0,5 bar auf diese Vormischung aufgesprüht. Im Verlauf des Granulierprozesses werden die Sprührate auf 12 g/min und die Zulufttemperatur auf 100 °C erhöht, wobei die Luftmenge auf max. 30 m³/h gesteigert wird. Die Produkttemperatur wird während des gesamten Verfahrens auf etwa 30-31 °C gehalten. Nach dem Auftrag von 310 g werden die Pellets bei einer max. Produkttemperatur von 57 °C getrocknet, anschließend auf etwa 30 °C abgekühlt und gesiebt.

### Beispiel 2: Herstellung von Farbstoffpellets mittels Extrudiertechnologie

### Farbstoffpelletvormischung

| | |
|---|---|
| 1896 g | 4-(2Hydroxyethylamino)-3-nitrophenol |
| 504 g | 2-((2-Hydroxyethyl)amino)-4,6-dinitro-phenol |
| 800 g | mikrokristalline Cellulose |
| 800 g | Corn Starch (Maisstärke) |

Die Farbstoffpelletvormischung wird in einem Vertikalgranulierer (Rotordrehzahl = etwa 150 U/min; Zerhackerdrehzahl = etwa 1000 U/min) 1 Minute lang vermischt und anschließend mittels einer Zweistoffdüse unter weiterem Mischen mit 2091 g einer 6 %igen wässrigen Hydroxypropylmethylcellulose-Lösung besprüht. Die so erhaltene Masse wird mittels eines Extruders Typ BR 200 (Drehzahl = 27 U/min; Sieb∅: 1,0 mm) bei einer Produkttemperatur von etwa 30 °C extrudiert. Anschließend wird das so erhaltene Material in einem Pelletizer Typ P 50 1 Minute lang bei 550 U/min verrundet und sodann in einem Glatt-Vertikalgranulierer bei einer Zulufttemperatur von 70 °C und einer Luftmenge von etwa 60-90 m³/h sowie einer max. Produkttemperatur von 51 °C getrocknet. In einem Glatt-Wirbelschichtgranulator und Coater werden 1500 g der getrockneten Farbstoffpellets bei einer Zulufttemperatur von etwa 50 °C und einer Luftmenge von 75 m³/h auf eine Produkttemperatur von 39-40 °C erwärmt. Sodann werden die Pellets bei einer Sprührate von 5 g/min und einem Sprühluftdruck von 2,5 bar mit einer 10 %igen wässrigen Hydroxypropylmethylcellulose-Lösung besprüht, wobei im Verlauf des Prozesses die Sprührate auf 8,5 g/min erhöht wird. Nach dem Aufbringen von 2215 g der Sprühlösung, entsprechend einem 14 %igen Feststoffauftrag, wird bei einer Produkttemperatur von max. 51 °C (Zulufttemperatur = etwa 70 °C) erneut getrocknet, anschließend auf etwa 27 °C abgekühlt und gesiebt.
[Alternativ können Trocknung und Coating bzw. Granulierung, Trocknung und Coating auch in einem gemeinsamen Arbeitsschritt erfolgen.]

### Beispiel 3: Herstellung von Farbstoffpellets mittels Extrudiertechnologie

### Farbstoffpelletvormischung

| | |
|---|---|
| 1411 g | 2,5-Diamino-toluol-sulfat |
| 636 g | 4-Amino-2-hydroxytoluol |
| 353 g | 2-Amino-4-(β-hydroxyethylamino)-anisol-sulfat |
| 794 g | Ascorbinsäure |
| 1058 g | Natriumsulfit |
| 800 g | Hydroxypropylcellulose |
| 1300 g | Maisstärke |

Die Herstellung erfolgt in Analogie zu Beispiel 2 jedoch wird eine 5,625 %ige wässrige Hydroxypropyl-methylcellulose-Lösung als Coating-Mittel verwendet.

### Beispiel 4: Herstellung von Farbstoffpellets im Top-Spray-Verfahren

### Farbstoffpelletvormischung

| | |
|---|---|
| 7,2 g | 5-Amino-2-methyl-phenol |
| 16,0 g | 2,5-Diamino-toluol-sulfat |
| 4,0 g | 2-Amino-4-(β-hydroxyethylamino)-anisol-sulfat |
| 3,0 g | Ascorbinsäure |
| 4,0 g | Natriumsulfit |
| 965,8 g | Hydrolyzed Corn Starch (Oligosaccharid) |

Die Farbstoffpelletvormischung wird in der in Beispiel 1 beschriebenen Weise mit 563 g einer 20%igen wässrigen Polyvinylpyrrolidon-Lösung pelletiert.

### Beispiel 5: Herstellung von Farbstoffpellets im Top Spray Verfahren

### Farbstoffpelletvormischung

| | |
|---|---|
| 38,6 g | 2,4-Diaminophenoxyethanol-Hydrochlorid |
| 47,1 g | N,N-Bis(2-hydroxyethyl)-p-phenylendiamin-Sulfat |
| 3,0 g | HC Blue No. 15 |
| 30,0 g | Ascorbinsäure |
| 10,0 g | Ethylendiaminotetraessigsäure-Dinatriumsalz |
| 500,0 g | Gummi Arabicum, 20 %ige Lösung in Wasser |

### Füllmaterial

| | |
|---|---|
| 771,3 g | Hydrogenated saccharides (Hauptbestandteil: 6-O-α-glucopyranolyl-D-sorbitol und 1-O-α-glucopyranolyl-D-mannitol) |

In einem Glatt-Wirbelschichtgranulator und Coater wird das Füllmaterial vorgelegt und bei einer Zulufttemperatur von 75 °C und einer Luftmenge von 55-65m³/h auf eine Produkttemperatur von etwa 34 °C erwärmt. Anschließend wird die wässrige Farbstoffpelletvormischung ("Sprühlösung") mit einer Anfangssprührate von 15-22 g/min und einem Sprühluftdruck von 1,2-1,4 bar auf das vorgelegte Füllmaterial gesprüht. Im Verlauf des Granulierprozeßes wird die Sprührate und die Zulufttemperatur konstant gehalten. Die Luftmenge wird je nach Farbstoffpelletvormischung auf maximal 100 m³/h gesteigert. Die Produkttemperatur wird je nach Farbstoffpelletvormischung während des gesamten Verfahrens zwischen 40 und 60 °C gehalten. Nach dem Aufbringen der Farbstoffpelletvormischung werden die Pellets bei einer maximalen Produkttemp. von 60 °C getrocknet, anschließend auf etwa 30 °C abkühlen gelassen und gesiebt.

### Beispiel 6: Herstellung von Farbstoffpellets im Top Spray Verfahren

### Farbstoffpelletvormischung

| | |
|---|---|
| 63,0 g | 1-Hydroxyethyl-4,5-diamino-pyrazol-Sulat |
| 32,5 g | 4-Amino-2-hydroxytoluol |
| 2,0 g | HC Red No. 10 / HC Red No. 11 (im Verhältnis 70/30) |
| 30,0 g | Ascorbinsäure |
| 10,0 g | Ethylendiaminotetraessigsäure-Dinatriumsalz |
| 500,0 g | Gummi Arabicum, 20 %ige Lösung in Wasser |

### Füllmaterial

| | |
|---|---|
| 762,5 g | Hydrogenated saccharides (Hauptbestandteil: 6-O-α-glucopyranolyl-D-sorbitol und 1-O-α-glucopyranolyl-D-mannitol) |

In einem Glatt-Wirbelschichtgranulator und Coater wird das Füllmaterial vorgelegt und bei einer Zulufttemperatur von 75 °C und einer Luftmenge von 55-65m³/h auf eine Produkttemperatur von etwa 34 °C erwärmt. Anschließend wird die wässrige Farbstoffpelletvormischung ("Sprühlösung") mit einer Anfangssprührate von 15-22 g/min und einem Sprühluftdruck von 1,2-1,4 bar auf das vorgelegte Füllmaterial gesprüht. Im Verlauf des Granulierprozeßes wird die Sprührate und die Zulufttemperatur konstant gehalten. Die Luftmenge wird je nach Farbstoffpelletvormischung auf maximal 100 m³/h gesteigert. Die Produkttemperatur wird je nach Farbstoffpelletvormischung während des gesamten Verfahrens zwischen 40 und 60 °C gehalten. Nach dem Aufbringen der Farbstoffpelletvormischung werden die Pellets bei einer maximalen Produkttemp. von 60 °C getrocknet, anschließend auf etwa 30 ° C abkühlen gelassen und gesiebt.

### Beispiel 7: Herstellung von Farbstoffpellets im Top Spray Verfahren

### Farbstoffpelletvormischung

| | |
|---|---|
| 12,0 g | 4-Amino-3-methyl-phenol |
| 12,0 g | 4-Amino-2-hydroxytoluol |
| 80,0 g | 4-Amino-6-chloro-4-nitrophenol |
| 30,0 g | Ascorbinsäure |
| 10,0 g | Ethylendiaminotetraessigsäure-Dinatriumsalz |
| 500,0 g | Gummi Arabicum, 20 %ige Lösung in Wasser |

### Füllmaterial

| | |
|---|---|
| 756,0 g | Hydrogenated saccharides (Hauptbestandteil: 6-O-α-glucopyranolyl-D-sorbitol und 1-O-α-glucopyranolyl-D-mannitol) |

In einem Glatt-Wirbelschichtgranulator und Coater wird das Füllmaterial vorgelegt und bei einer Zulufttemperatur von 75 °C und einer Luftmenge von 55-65m³/h auf eine Produkttemperatur von etwa 34 °C erwärmt.
Anschließend wird die wässrige Farbstoffpelletvormischung ("Sprühlösung") mit einer Anfangssprührate von 15-22 g/min und einem Sprühluftdruck von 1,2-1,4 bar auf das vorgelegte Füllmaterial gesprüht. Im Verlauf des Granulierprozeßes wird die Sprührate und die Zulufttemperatur konstant gehalten. Die Luftmenge wird je nach Farbstoffpelletvormischung auf maximal 100 m³/h gesteigert. Die Produkttemperatur wird je nach Farbstoffpelletvormischung während des gesamten Verfahrens zwischen 40 und 60 °C gehalten. Nach dem Aufbringen der Farbstoffpelletvormischung werden die Pellets bei einer maximalen Produkttemp. von 60 °C getrocknet, anschließend auf etwa 30 ° C abkühlen gelassen und gesiebt.

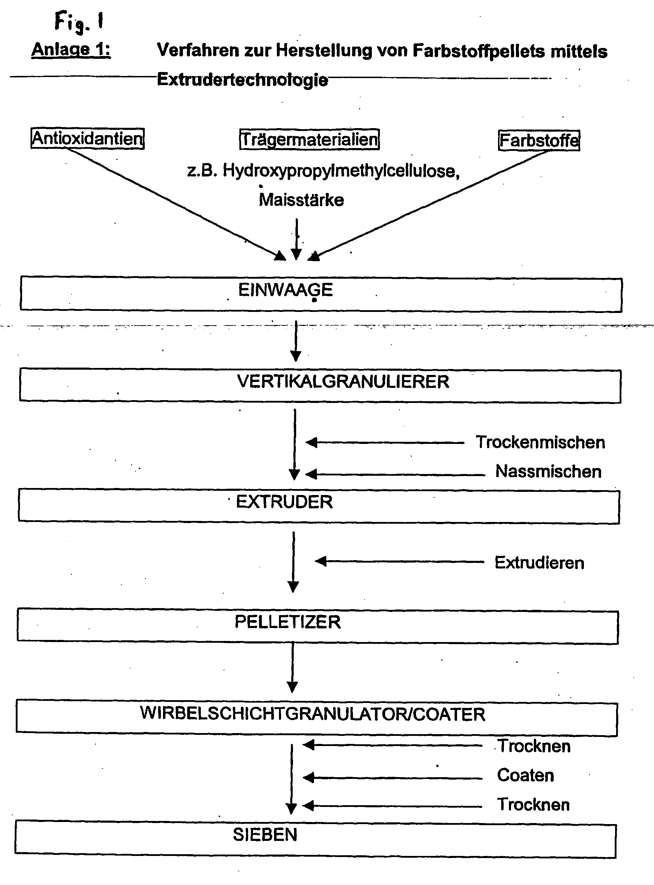

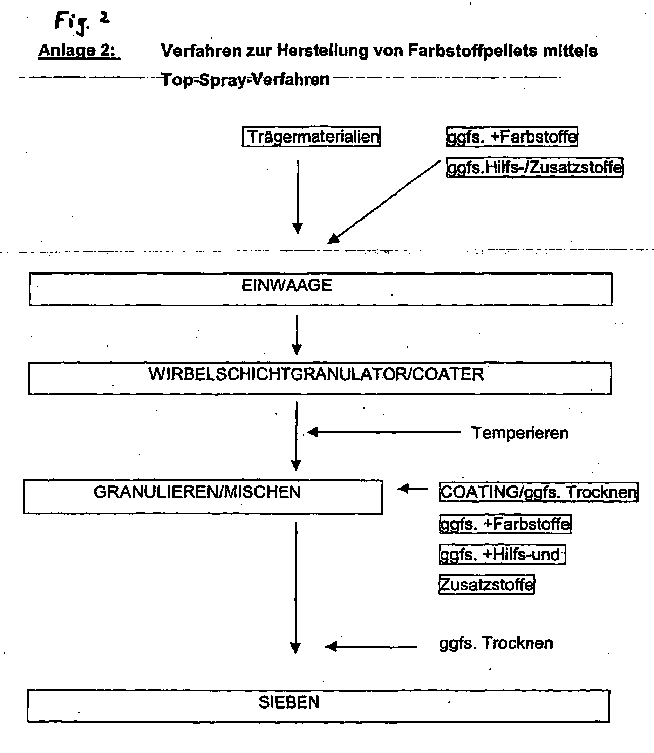

## Patentansprüche

1. Mehrkomponenten-Kit zum Färben von Keratinfasern, **dadurch gekennzeichnet, dass** es aus (i) einer Farbstoffträgermasse (A), welche frei ist von Farbstoffen und Farbstoffvorstufen; (ii) mehreren Zusammensetzungen (B) bestehend aus farbstoffhaltigen Pellets, welche durch homogenes Vermischen eines mindestens einen natürlichen und/oder- synthetischen Farbstoff enthaltenden Ausgangsstoffes mit einem geeigneten Trägermaterial und anschließende Beschichtung mit einem geeigneten Verkapselungsmaterial erhalten werden und mindestens ein Oxidationsfarbstoffvorprodukt und/oder mindestens einen Direktfarbstoff sowie gegebenenfalls mindestens eine keratinaufhellende bzw. bleichende Substanz enthalten; und gegebenenfalls (iii) einem geeigneten Oxidationsmittel (C) besteht.

2. Mehrkomponenten-Kit zum Färben von Keratinfasern, **dadurch gekennzeichnet, dass** es aus (i) einer Farbstoffträgermasse (A), welche frei ist von Farbstoffen und Farbstoffvorstufen; (ii) mehreren Zusammensetzungen (B) bestehend aus farbstoffhaltigen Pellets, welche durch Beschichtung eines geeigneten Trägermaterials mit einer Mischung aus mindestens einem natürlichen und/oder synthetischen Farbstoff und mindestens einem geeigneten Verkapselungsmaterial erhalten werden und mindestens ein Oxidationsfarbstoffvorprodukt und/oder mindestens einen Direktfarbstoff sowie ggfs. mindestens eine keratinaufhellende bzw. bleichende Substanz enthalten; und ggfs. (iii) einem geeigneten Oxidationsmittel (C) besteht.

3. Mehrkomponenten-Kit nach Anspruch 1 oder 2, **dadurch gekennzeichnet, dass** bei dem Farbstoffpellet das Trägermaterial ausgewählt ist aus Gummi Arabicum, Polyvinylpyrrolidonen, Dextrose, Oligosacchariden, mikrokristallinen Cellulose-Derivaten, physikalisch beziehungsweise chemisch modifizierten Stärken oder Stärkederivaten, synthetischem Calciumsilicat, Kieselgur und Siliziumdioxid.

4. Mehrkomponenten-Kit nach einem der Ansprüche 1 bis 3, **dadurch gekennzeichnet, dass** bei dem Farbstoffpellet das Trägermaterial ausgewählt ist aus Gummi Arabicum, Polyvinylpyrrolidon, Dextrose, Oligosacchariden, Hydroxypropylmethylcellulose, Methylcellulose, Hydroxyethylcellulose, Hydroxypropylcellulose, Nonoxynolhydroxyethylcellulose, Cetylhydroxyethylcellulose und mit Propylenoxid modifizierten nichtionischen Stärkederivaten.

5. Mehrkomponenten-Kit nach einem der Ansprüche 1 bis 4, **dadurch gekennzeichnet, dass** bei dem Farbstoffpellet das Verkapselungsmaterial ausgewählt ist aus Gummi Arabicum, Cellulose-Derivaten, Polyethylen-Dispersionen, Polyacrylsäuren, Polyvinylalkoholen, Polyvinylpyrrolidonen, Polycarbonaten, Polyestern, Polyamiden oder natürlichen Filmbildnern.

6. Verfahren zum Färben oder gleichzeitigen Färben und Aufhellen von Keratinfasern, bei dem auf die Keratinfaser ein unmittelbar vor der Anwendung individuell hergestelltes Gemisch aus den Zusammensetzungen (A) und (B) sowie ggfs. (C) aus einem Mehrkomponenten Kit gemäß einem der Ansprüche1 bis 5 aufgetragen wird.

7. Verfahren nach Anspruch 6, **dadurch gekennzeichnet, dass** die Komponente (A) in einer Menge von 10 bis 120 g, die Komponente (B) in einer Menge von 0,1 bis 20 g und die Komponente (C) in einer Menge von 10 bis 120 g eingesetzt wird.

8. Verfahren nach Anspruch 6 oder 7, **dadurch gekennzeichnet, dass** das Verhältnis von (A) zu (B) gleich 1000: 1 bis 2:1 ist und das Verhältnis von (C) zu (A) gleich 3:1 bis 1:3 ist.

9. Verwendung eines Mehrkomponenten-Kits bestehend aus einer Kombination aus (i) einer farbstofffreien Farbstoffträgermasse (A) und (ii) mehreren Zusammensetzungen (B) bestehend aus farbstoffhaltigen Pellets, welche durch homogenes Vermischen eines mindestens einen natürlichen und/oder synthetischen Farbstoff enthaltenden Ausgangsstoffes mit einem geeigneten Trägermaterial und anschließende Beschichtung mit einem geeigneten Verkapselungsmaterial erhalten werden und mindestens ein Oxidationsfarbstoffvorprodukt und/oder mindestens einen Direktfarbstoff sowie ggfs. mindestens eine keratinaufhellende bzw. bleichende Substanz enthalten, sowie (iii) ggfs. einem Oxidationsmittel (C), zur individuellen Herstellung von Färbemitteln für Keratinfasern unmittelbar vor der Färbung der Keratinfasern.

10. Verwendung eines Mehrkomponenten-Kits bestehend aus einer Kombination aus (i) einer farbstofffreien Farbstoffträgermasse (A) und (ii) mehreren Zusammensetzungen (B) bestehend aus farbstoffhaltigen Pellets, welche durch Beschichtung eines geeigneten Trägermaterials mit einer Mischung aus mindestens einem natürlichen und/oder synthetischen Farbstoff und mindestens eines geeigneten Verkapselungsmaterials erhalten werden, und mindestens ein Oxidationsfarbstoffvorprodukt und/oder mindestens einen Direktfarbstoff sowie ggfs. mindestens eine keratinauthellende bzw. bleichende Substanz enthalten, sowie (iii) ggfs. einem Oxidationsmittel (C), zur individuellen Herstellung von Färbemitteln für Keratinfasern unmittelbar vor der Färbung der Keratinfasern.

## Claims

1. Multicomponent kit for dyeing keratin fibres, **characterized in that** it consists of (i) a dye carrier mass (A) which is free from dyes and dye precursors; (ii) several compositions (B) consisting of dye-containing pellets which are obtained by homogeneous mixing of a starting material comprising at least one natural and/or synthetic dye with a suitable carrier material and subsequent coating with a suitable encapsulation material, and at least one oxidation dye precursor and/or at least one direct dye and optionally at least one keratin-lightening or bleaching substance; and optionally (iii) a suitable oxidizing agent (C).

2. Multicomponent kit for dyeing keratin fibres, **characterized in that** it consists of (i) a dye carrier mass (A) which is free from dyes and dye precursors; (ii) several compositions (B) consisting of dye-containing pellets which are obtained by coating a suitable carrier material with a mixture of at least one natural and/or synthetic dye and at least one suitable encapsulation material, and at least one oxidation dye precursor and/or at least one direct dye, and optionally at least one keratin-lightening or bleaching substance; and optionally (iii) a suitable oxidizing agent (C).

3. Multicomponent kit according to Claim 1 or 2, **characterized in that,** in the case of the dye pellet, the carrier material is chosen from gum arabic, polyvinylpyrrolidones, dextrose, oligosaccharides, microcrystalline cellulose derivatives, physically or chemically modified starches or starch derivatives, synthetic calcium silicate, kieselguhr, silicon dioxide.

4. Multicomponent kit according to one of Claims 1 to 3, **characterized in that,** in the case of the dye pellet, the carrier material is chosen from gum arabic, polyvinylpyrrolidone, dextrose, oligosaccharides, hydroxypropylmethylcellulose, methylcellulose, hydroxyethylcellulose, hydroxypropylcellulose, nonoxynolhydroxyethylcellulose and cetylhydroxyethylcellulose and nonionic starch derivatives modified with propylene oxide.

5. Multicomponent kit according to one of Claims 1 to 4, **characterized in that,** in the case of the dye pellet, the encapsulation material is chosen from gum arabic, cellulose derivatives, polyethylene dispersions, polyacrylic acids, polyvinyl alcohols, polyvinylpyrrolidones, polycarbonates, polyesters, polyamides or natural film formers.

6. Method for dyeing or simultaneously dyeing and lightening keratin fibers, in which a mixture of the compositions (A) and (B) and optionally (C) from a multicomponent kit according to one of Claims 1 to 5 prepared individually directly prior to use is applied to the keratin fibres.

7. Method according to Claim 6, **characterized in that** component (A) is used in an amount of from 10 to 120 g, component (B) is used in an amount of from 0.1 to 20 g and component (C) is used in an amount of from 10 to 120 g.

8. Method according to Claim 6 or 7, **characterized in that** the ratio of (A) to (B) is 1000:1 to 2:1 and the ratio of (C) to (A) is 3:1 to 1:3.

9. Use of a multicomponent kit consisting of a combination of (i) a dye-free dye carrier mass (A) and (ii) several compositions (B) consisting of dye-containing pellets which are obtained by homogeneous mixing of a starting material comprising at least one natural and/or synthetic dye with a suitable carrier material and subsequent coating with a suitable encapsulation material, and at least one oxidation dye precursor and/or at least one direct dye, and optionally at least one keratin-lightening or bleaching substance, and also (iii) optionally an oxidizing agent (C) for the individual preparation of colourants for keratin fibres directly prior to dyeing the keratin fibres.

10. Use of a multicomponent kit consisting of a combination of (i) a dye-free dye carrier mass (A) and (ii) several compositions (B) consisting of dye-containing pellets which are obtained by coating a suitable carrier material with a mixture of at least one natural and/or synthetic dye and at least one suitable encapsulation material, and at least one oxidation dye precursor and/or at least one direct dye, and optionally at least one keratin-lightening or bleaching substance, and (iii) optionally an oxidizing agent (C) for the individual preparation of colourants for keratin fibres directly prior to dyeing the keratin fibres.

## Revendications

1. Nécessaire à plusieurs composants pour la teinture de fibres de kératine, **caractérisé en ce qu**'il est constitué de (i) une matière porteuse de colorant (A) qui est exempte de colorants et de précurseurs de colorants ; (ii) plusieurs compositions (B) consistant en des granules contenant un colorant, qui sont obtenus par mélange homogène d'un produit de départ, contenant au moins un colorant naturel et/ou un colorant synthétique, avec un matériau de support approprié et enrobage subséquent avec un matériau d'encapsulation approprié, et contiennent au moins un précurseur de colorant d'oxydation et/ou au moins un colorant direct ainsi qu'éventuellement au moins une substance décolorante ou éclaircissant la kératine ; et éventuellement (iii) un oxydant (C) approprié.

2. Nécessaire à plusieurs composants pour la teinture de fibres de kératine, **caractérisé en ce qu**'il est constitué de (i) une matière porteuse de colorant (A) qui est exempte de colorants et de précurseurs de colorants ; (ii) plusieurs compositions (B) consistant en des granules contenant un colorant, qui sont obtenus par enrobage d'un matériau de support approprié avec un mélange d'au moins un colorant naturel et/ou un colorant synthétique et d'au moins un matériau d'encapsulation approprié, et contiennent au moins un précurseur de colorant d'oxydation et/ou au moins un colorant direct ainsi qu'éventuellement au moins une substance décolorante ou éclaircissant la kératine ; et éventuellement (iii) un oxydant (C) approprié.

3. Nécessaire à plusieurs composants selon la revendication 1 ou 2, **caractérisé en ce que** dans le granule contenant un colorant le matériau de support est choisi parmi la gomme arabique, les polyvinylpyrrolidones, le D-glucose, des oligosaccharides, des dérivés de cellulose microcristalline, des amidons ou dérivés d'amidon modifiés physiquement ou chimiquement, le silicate de calcium synthétique, le kieselguhr, le dioxyde de silicium.

4. Nécessaire à plusieurs composants selon l'une quelconque des revendications 1 à 3, **caractérisé en ce que** dans le granule contenant un colorant le matériau de support est choisi parmi la gomme arabique, les polyvinylpyrrolidones, le D-glucose, des oligosaccharides, l'hydroxypropylméthylcellulose, la méthylcellulose, l'hydroxyéthylcellulose, l'hydroxypropylcellulose, la nonoxynolhydroxyéthylcellulose et la cétyl-hydroxyéthylcellulose et des dérivés d'amidon non ioniques modifiés avec de l'oxyde de propylène.

5. Nécessaire à plusieurs composants selon l'une quelconque des revendications 1 à 4, **caractérisé en ce que** dans le granule contenant un colorant le matériau d'encapsulation est choisi parmi la gomme arabique, des dérivés de cellulose, des dispersions de polyéthylène, les poly(acide acrylique)s, les poly(alcool vinylique)s, les polyvinylpyrrolidones, les polycarbonates, les polyesters, les polyamides ou des agents filmogènes naturels.

6. Procédé pour la teinture ou la teinture et l'éclaircissement simultanés de fibres de kératine, dans lequel on applique sur les fibres de kératine un mélange, préparé individuellement, immédiatement avant l'emploi, à partir des compositions (A) et (B) ainsi qu'éventuellement (C) du nécessaire à plusieurs composants selon l'une quelconque des revendications 1 à 5.

7. Procédé selon la revendication 6, **caractérisé en ce qu**'on utilise le composant (A) en une quantité de 10 à 120 g, le composant (B) en une quantité de 0,1 à 20 g et le composant (C) en une quantité de 10 à 120 g.

8. Procédé selon la revendication 6 ou 7, **caractérisé en ce que** le rapport de (A) à (B) va de 1000:1 à 2:1 et le rapport de (C) à (A) va de 3:1 à 1:3.

9. Utilisation d'un nécessaire à plusieurs composants consistant en une association de (i) une matière porteuse de colorant (A) exempte de colorant et (ii) plusieurs compositions (B) consistant en des granules contenant un colorant, qui sont obtenus par mélange homogène d'un produit de départ, contenant au moins un colorant naturel et/ou un colorant synthétique, avec un matériau de support approprié et enrobage subséquent avec un matériau d'encapsulation approprié, et contiennent au moins un précurseur de colorant d'oxydation et/ou au moins un colorant direct ainsi qu'éventuellement au moins une substance décolorante ou éclaircissant la kératine ; ainsi que (iii) éventuellement un oxydant (C), pour la préparation individuelle de compositions de teinture pour fibres de kératine immédiatement avant la teinture des fibres de kératine.

10. Utilisation d'un nécessaire à plusieurs composants consistant en une association de (i) une matière porteuse de colorant (A) exempte de colorant et (ii) plusieurs compositions (B) consistant en des granules contenant un colorant, qui sont obtenus par enrobage d'un matériau de support approprié avec un mélange d'au moins un colorant naturel et/ou un colorant synthétique et d'au moins un matériau d'encapsulation approprié, et contiennent au moins un précurseur de colorant d'oxydation et/ou au moins un colorant direct ainsi qu'éventuellement au moins une substance décolorante ou éclaircissant la kératine ; ainsi que (iii) éventuellement un oxydant (C), pour la préparation individuelle de compositions de teinture pour fibres de kératine immédiatement avant la teinture des fibres de kératine.
